(19) Europäisches Patentamt
European Patent Office
Office européen des brevets

(11) Publication number: **0 498 222 A1**

(12) **EUROPEAN PATENT APPLICATION**

(21) Application number: **92101084.9**

(51) Int. Cl.$^5$: **C07K 7/64**

(22) Date of filing: **23.01.92**

The applicant has subsequently filed a sequence listing and declared, that it includes no new matter.

(30) Priority: **24.01.91 JP 25374/91**

(43) Date of publication of application: **12.08.92 Bulletin 92/33**

(84) Designated Contracting States: **BE CH DE ES FR GB LI NL**

(71) Applicant: **SUMITOMO CHEMICAL COMPANY LIMITED**
**5-33, Kitahama 4-chome, Chuo-ku**
**Osaka-shi, Osaka 541(JP)**

(72) Inventor: **Chino, Haruo**
**Kita 3-74, Hondori 18-chome Shiraishi-ku**
**Sapporo-shi, Hokkaido(JP)**
Inventor: **Hayakawa, Yoichi**
**2-8-4-25-308, Miyanomori Chuo-ku**
**Sapporo-shi, Hokkaido(JP)**

(74) Representative: **Vossius & Partner**
**Siebertstrasse 4 P.O. Box 86 07 67**
**W-8000 München 86(DE)**

(54) **Novel peptides.**

(57) A peptide of the general formula [I]:

```
Glu-Asn-Phe-Ser-Gly-Gly-Cys-Val-Ala-Gly-
Tyr-Met-Arg-Thr-Pro-Asp-Gly-Arg-Cys-Lys-     [I]
Pro-Thr-Phe-Tyr-Gln-R
```

wherein R is an amino or hydroxyl group.

EP 0 498 222 A1

The present invention relates to peptides of the formula [I] (hereinafter referred to as peptide [I]):

Glu-Asn-Phe-Ser-Gly-Gly-Cys-Val-Ala-Gly-

Tyr-Met-Arg-Thr-Pro-Asp-Gly-Arg-Cys-Lys- [I]

Pro-Thr-Phe-Tyr-Gln-R

wherein R is an amino group or hydroxy group having insect pest controlling activity, i.e., insect development controlling or insecticidal activity.

Various insect function or development controlling peptides are already known. Thus, for instance, PTTH [Nagasawa et al., Proceedings of the National Academy of Sciences of the United States of America, 83, 5840-5843 (1986)], EH [Truman et al., Nature, 291, 70-71 (1981)] and AKHI [Stone et al., Nature, 263, 207-211 (1976)] have been isolated and their functions and other features have been revealed.

The present inventors found out that certain novel peptides can markedly inhibit the development of or cause death to insects when the insects themselves are treated with very small amounts of said peptides. This finding has now led to the present invention.

The present inventors found out that a peptide (hereinafter referred to as peptide I-a) of the formula [I-a]:

Glu-Asn-Phe-Ser-Gly-Gly-Cys-Val-Ala-Gly-

Tyr-Met-Arg-Thr-Pro-Asp-Gly-Arg-Cys-Lys- [I-a]

Pro-Thr-Phe-Tyr-Gln-$NH_2$

which is obtainable from the plasma of last instar larvae of the armyworm Pseudaletia separata parasitized with the parasitoid wasp Apanteles karivai and has JH (juvenile hormone) esterase repressing activity and has an excellent insect development controlling activity.

The present inventors further found out that a peptide (hereinafter referred to as peptide I-b) of the formula [I-b]:

Glu-Asn-Phe-Ser-Gly-Gly-Cys-Val-Ala-Gly-

Tyr-Met-Arg-Thr-Pro-Asp-Gly-Arg-Cys-Lys- [I-b]

Pro-Thr-Phe-Tyr-Gln-OH

which is similar in structure to peptide I-a has excellent insecticidal activity. The present invention has been completed based on the above findings.

Fig. 1 graphically shows the time profile of reversed phase chromatography of roughly purified peptide I-a on a cyanopropyl-derived silica HPLC column.

Fig. 2 graphically shows the time profile of reversed phase chromatography of the bioactive fraction indicated by a bold line

( ▬ )

in Fig. 1 on a cyanopropyl-derived silica HPLC column.

Fig. 3 graphically shows the time profile of reversed phase chromatography of the bioactive fraction indicated by a bold line in

( ▬ )

Fig. 2 on a cyanopropyl-derived silica HPLC column. The fraction indicated by a bold line

( ▬ )

in this figure is the purified peptide I-a.

Fig. 4 graphically shows the body weight change in last instar larvae of Pseudaletia separata injected with peptide I-a. The open circles (○) indicate the body weights of the last instar larvae injected with 7 picomoles of peptide I-a on day 0, day 1 and day 2 while the closed circles (●) indicate the body weights of last instar larvae injected with Ringer's solution on day 0, day 1 and day 2 of the last instar.

Fig. 5 graphically shows the results of JH esterase activity assay of hemolymph samples collected, on day 3, from last instar larvae of Pseudaletia separata injected with pre-determined doses of peptide I-a on day 1 and day 2 of the last instar.

Peptide I-a of the present invention can be obtained in a purified form, for example by extracting, with 25% ethanol, the hemolymph of last instar larvae of Pseudaletia separata parasitized with the Apanteles kariyai and subjecting the extract to gel permeation chromatography and reversed phase high performance liquid chromatography.

In more detail , last (6th) instar larvae of Pseudaletia separata are parasitized with Apanteles kariyai by exposing them to female wasps for allowing oviposition by the latter. The larvae are reared on an artificial diet for 4 to 6 days. Then the hemolymph is collected from the larvae and, after addition of chilled ethanol (-20 ° C) to a final concentration of 25%, subjected to lyophilization.

The lyophilization product is roughly purified by reversed phase column chromatography, and the roughly purified fraction is concentrated and subjected to gel permeation high performance liquid chromatography and reversed phase high performance liquid chromatography, whereby purified peptide I-a can be obtained with JH esterase activity repressing activity as a marker.

The following example is further illustrative of the present invention but are by no means limitative of the scope thereof.

In the Examples, day X of the last instar is simply referred to as day X.

EXAMPLE 1

Purification of peptide I-a

Last instar larvae of the Pseudaletia separata were parasitized with eggs of the wasp Apanteles kariyai on day 1 and then reared on an artificial diet.

The hemolymph of parasitized day 4 to 7 last instar larvae (3-6 days after parasitization) was collected into a chilled polypropylene test tube added 500 µl of 0.2 M phosphate buffer (pH 6.0) containing 0.05% phenylthiourea by the flushing out method with an injection of about 0.1 ml of 0.1 M phosphate buffer containing 150 mM KCl and 10 mM EDTA into each hemocoele. The hemolymph collected was immediately centrifuged at 4 ° C for 5 minutes at 500 x g and the supernatant was collected.

The supernatant thus collected was mixed with cold ethanol (-20 ° C; final concentration = 25%) and, following centrifugation (4 ° C, 15 minutes, 20,000 x g), the supernatant thus obtained was concentrated by lyophilization and applied to a 3-ml volume of $C_{18}$ extraction column (J. T. Baker Chemical Co.) equilibrated with 6 ml of distilled water.

The column was washed with 5 ml of distilled water and then eluted with 2.5 ml of 30% acetonitrile. The eluate was concentrated under a stream of nitrogen and subjected to gel permeation chromatography on Superose 6 HR 10/30 column (Pharmacia LKB Biotechnology Inc.) equilibrated with 12.5 mM phosphate buffer (pH 6.6).

The bioactive fractions was collected and further applied on a reversed phase $C_8$ HPLC column (product of Yamamura Chemical Research Institute; 4.6 mm x 250 mm) by elution with a $CH_3CN$ gradient (elevated from 18 to 40%) in 0.1% $CF_3COOH/H_2O$.

The bioactive fraction was again collected and rechromatographed using a reversed phase cyanopropyl-derived silica HPLC column (product of Yamamura Chemical Research Institute; 4.6 mm x 250 mm), elution being made with a gradient of $CH_3CN$ (from 15 to 25%) in 0.1% $CF_3COOH/H_2O$. The result is shown in Fig. 1.

Fig. 1 graphically shows the time profile of reversed phase chromatography of roughly purified peptide I-a on a cyanopropyl-derived silica HPLC column.

The active fraction indicated by a bold line in Fig. 1 was reapplied to the same column as mentioned above and eluted with a gradient of 18 to 25% $CH_3CN$ in 0.05% $CF_3CF_2CF_2COOH/H_2O$. The result is shown in Fig. 2.

Fig. 2 graphically shows the time profile of reversed phase chromatography of the bioactive fraction indicated by a bold line

(▬)

in Fig. 1 on a cyanopropyl-derived silica HPLC column.

The active fraction indicated by a bold line in Fig. 2 was further applied to the same column as mentioned above and then eluted using a gradient of 20 to 25% $CH_3CN$ in 0.075% $CF_3CF_2CF_2COOH/H_2O$. The result is shown in Fig. 3.

The fraction indicated by a bold line

(▬)

in Fig. 3 is the final purified fraction.

Fig. 3 graphically shows the time profile of reversed phase chromatography of the bioactive fraction indicated by a bold line

(▬)

in Fig. 2 on a cyanopropyl-derived silica HPLC column. The fraction indicated by a bold line

(▬)

in this figure is the purified peptide I-a.

The degree of purification, recovery and other data obtained in the above purification process are summarized below in Table 1.

Table 1

| Sample from | Total volume (ml) | Protein (µg/ml) | Total activity (units) | Specific activity (units/µg) | Purification | Recovery (%) |
|---|---|---|---|---|---|---|
| Hemolymph | 21.4 | 18,100 | 1,698 | 0.00438 | 1.00 | 100 |
| Ethanol extract | 26.8 | 1,490 | 547 | 0.0137 | 3.15 | 32.2 |
| $C_{18}$ column | 3.0 | 179 | 353 | 0.658 | 150 | 20.8 |
| Superose 6 HR | 3.2 | 86.9 | 303 | 1.09 | 249 | 17.8 |
| Reversed phase $C_8$ column | 2.4 | 21.2 | 248 | 4.89 | 1,120 | 14.6 |
| Cyanopropyl column (I) | 1.2 | 4.59 | 74.9 | 13.6 | 3,110 | 4.41 |
| Cyanopropyl column (II) | 0.52 | 3.67 | 48.9 | 25.6 | 5,840 | 2.88 |
| Cyanopropyl column (III) | 0.40 | 2.75 | 28.8 | 26.2 | 5,980 | 1.70 |

Furthermore, peptide [I] of the present invention can be produced by those synthetic methods that are generally known in the field of peptide chemistry [cf. e.g. Houben-Weyl, Methoden der Organischen Chemie (Methods of Organic Chemistry), volume 15/2], preferably by known techniques of solid phase peptide synthesis described, for example, in B. Merrifield, J. Am. Chem. Soc., 85, 2149 (1963) or R. C. Sheppard, Int. J. Peptide Protein Res., 21, 118 (1983), or techniques equivalent thereto.

List of abbreviations

The abbreviations used for amino acids are those three-letter codes that are described, for example, in European Journal of Biochemistry, 138, 9 (1984) and conventional in the field of peptide chemistry. The following is further used as abbreviations for chemical compounds or protective groups.

| | |
|---|---|
| Acm | acetamidomethyl |
| Boc | tert-butoxycarbonyl |
| But | tert-butyl |
| Bzl | benzyl |
| Cl-z | 4-chlorobenzyloxycarbonyl |
| DMF | dimethylformamide |
| Fmoc | 9-fluorenylmethoxycarbonyl |
| Me | methyl |
| 4-Mebzl | 4-methylbenzyl |
| Mtr | 4-methoxy-2,3,6-trimethylphenylsulfonyl |
| Mts | mesitylene-2-sulfonyl |
| NMP | N-methylpyrrolidone |
| Pmc | 2,2,5,7,8-pentamethylchroman-6-sulfonyl |
| TFA | trifluoroacetic acid |
| Tic | 1,2,3,4-tetrahydroisoquinolin-3-ylcarbonyl |
| Trt | trityl |

Thus, for example, urethane-forming protective groups, such as tert-butoxycarbonyl (Boc) and fluorenylmethoxycarbonyl (Fmoc), may be used as $\alpha$-amino-protective groups of peptide synthesis of the present peptide [I].

When necessary for the prevention of side reactions or for the synthesis of special peptides, the side chain functional groups of amino acids may further be protected with appropriate protective groups (cf. e.g., T. W. Greene, Protective Groups in Organic Synthesis).

In such cases, the following protected amino acids are preferred in most instances: Arg(Tos), Arg(Mts), Arg(Mtr), Arg(PMC), Asp(OBzl), Asp(OBut), Cys(4-MeBzl), Cys(Acm), Cys(SBut), Glu(OBzl), Glu(OBut), Lys-(Cl-z), Lys(Boc), Met(O), Ser(Bzl), Ser(But), Thr(Bzl), Thr(But), Tyr(Br-z), Tyr(Bzl) and Tyr(But).

The solid-phase polymerization starts with coupling of a protected amino acid for constituting the C terminus of the peptide to an appropriate resin. Starting materials of this type can be prepared by coupling the protected amino acid to a chloromethyl-, hydroxymethyl-, benzhydrylamino (BHA)- or methylbenzhydrylamino (MBHA)-modified polystyrene or polyacrylamide resin via ester or amide bonding.

The resin to be used as a supporting material can be obtained commercially. When peptides having an amide group at the C-terminus are desired, BHA and/or MBHA resin are generally used.

Removal of side chain protective groups is performed later using an appropriate reagent.

The amino protective group on the amino acid coupled to the resin is eliminated using an appropriate reagent, such as trifluoroacetic acid in methylene chloride in the case of Boc protective group or 20% piperidine in dimethylformamide in the case of Fmoc protective group. Unprotected amino acids are then subjected to coupling reaction in the desired order. Protective groups of each N-terminally protected intermediate peptide-resin are removed with a reagent such as mentioned above prior to coupling with the next amino acid derivative.

All activating reagents that are generally used for peptide synthesis, in particular carbodiimides such as N,N'-dicyclohexylcarbodiimide, N,N'-diisopropylcarbodiimide and N-ethyl-N'-(3-dimethylaminopropyl)-carbodiimide, can be used as coupling reagents [cf. e.g. Houben-Weyl, Methoden der Organischen Chemie (Methods of Organic Chemistry), volume 15/2].

In this case, the coupling reaction can be carried out directly by adding an amino acid derivative and an activating reagent, if necessary together with a racemization inhibiting additive such as 1-hydroxybenzotriazole (HOBt) [W. Konig, R. Geiger, Chem. Ber., 103, 708 (1970)] or 3-hydroxy-4-oxo-3,4-dihydrobenzotriazine (HOObt) [W. Konig, R. Geiger, Chem. Ber., 103, 2054 (1970)], to the resin. It is also possible to separately convert each amino acid derivative into a symmetric acid anhydride or an HOBt or HOObt ester for preliminary activation and add a solution of the activated substance in an appropriate solvent to the peptide-resin ready for coupling.

The coupling or activation of amino acid derivatives using one of the activating reagents mentioned above can be carried out in dimethylformamide, N-methylpyrrolidone or methylene chloride, or a mixed solvent composed of these.

Each activated amino acid derivative is conventionally used in 1.5- to 4-fold excess. In case of

incomplete coupling, the coupling reaction should be repeated without removing a protective group of the α-amino group of the peptide-resin which is otherwise necessary for coupling of the next amino acid.

The desired peptide synthesized in the above manner can be splitted off from the resin using such a reagent as liquified hydrogen fluoride (suitably when the peptide is produced by the Boc method) or tri fluoroacetic acid (suitably when the peptide is produced by the Fmoc method). Such reagent splits off not only the peptide from the resin but also other side chain protective groups remaining on respective amino acid derivative residues from the peptide.

In addition, when a BHA or MBHA resin is used in the above process, the peptide is obtained in the form of a free acid. When a BHA or MBHA resin is used and hydrogen fluoride or trifluoromethanesulfonic acid is used for splitting off the peptide from the resin, the peptide is obtained in the form of an acid amide.

An alternative method of peptide amide production is described, for example, in Japanese Unexamined Patent Publication No. 63-267748.

The peptide amide can be splitted off from the resin by treatment with an acid which is medium in strength and generally used in peptide synthesis (e.g. trifluoroacetic acid).

Cation entrainer substances which are conventional in solid-phase synthesis, for example phenol, cresol, thiocresol, anisole, thioanisole, ethanedithiol, dimethyl sulfide and ethyl methyl sulfide, are added either singly or in the form of a mixture of two or more of these adjuvants.

In this case, the adjuvants may be used with trifluoroacetic acid or an appropriate solvent such as methylene chloride.

The peptides of the present invention can preferably be synthesized employing the solid-phase technology and the tactics using two common protective groups.

The synthesis is performed using an Applied Biosystems model 430A automated peptide synthesizer, for instance, with Boc or Fmoc as a protective group for temporary blocking of the α-amino group.

When Boc is used as a protective group, the synthetic cycles preliminarily programmed by the manufacturer of the apparatus can be applied for the synthesis.

The synthesis of peptides having a free carboxyl group at the C terminus is performed on a (hydroxymethyl)phenylacetamidomethylstyrene resin [R. B. Merrifield, J. Org. Chem., 43, 2845 (1978)] modified or bound with the corresponding Boc amino acid available from Applied Biosystems, for instance.

The MBHA resin available from Applied Biosystems can be used for producing peptide amides. N,N'-dicyclohexylcarbodiimide or N,N'-diisopropylcarbodiimide is used as an activating reagent.

The activation can be carried out in $CH_2Cl_2$, a $CH_2Cl_2$-DMF mixture or NMP to give the corresponding symmetric acid anhydride, HOBt ester or HOObt ester.

For coupling, each activated amino acid derivative is used in an amount of 2 to 4 equivalents. If the coupling should be incomplete, the reaction should be repeated.

When Fmoc is used for temporary protection of the α-amino group, a model 430A automated peptide synthesizer produced by Applied Biosystems and a program for synthesis devised by the present inventors themselves can be used.

The synthesis is carried out on a p-benzyloxy benzyl alcohol resin [S. Wang, J. Am. Chem. Soc., 95, 1328 (1973); available from Bachem] esterified by a per se known method (E. Atherton et al., J. Chem. Soc. Chem. Comm., 1981, 336) using an appropriate amino acid.

The activation of amino acid derivatives as HOBt or HOObt esters may be carried out directly in an amino acid cartridge provided by the manufacturer of the apparatus by adding a solution of diisopropylcarbodiimide in DMF to a mixture of a weighed amount of an amino acid derivative and a weighed amount of HOBt or HOObt.

The Fmoc-amino acid-OObt esters may also be used in the same manner as described in Japanese Unexamined Patent Publication No. 62-286978.

The protective group Fmoc can be eliminated in the reaction vessel using a 20% solution of piperidine in DMF.

The reactive amino acid derivatives are used each in an amount 1.5 to 2.5 equivalents in excess. If the coupling should be incomplete, the reaction should be repeated as in the Boc process.

The following examples are further illustrative of the present invention but are by no means limitative of the scope thereof.

EXAMPLE 2

Synthesis of peptide I-a

Fmoc-Glu-OPfp (1 mmol) was reacted with a coupling group-modified aminomethyl resin having the

structural formula:

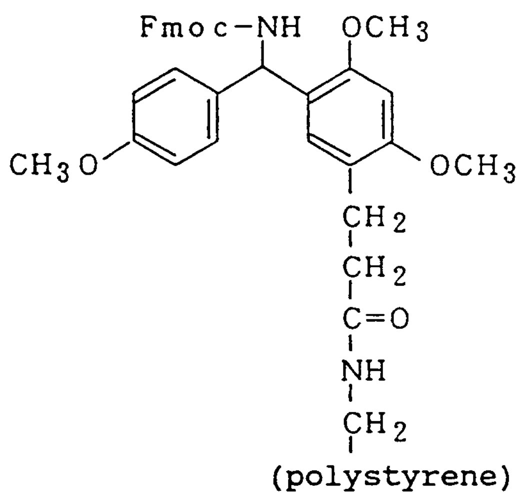

wherein Fmoc is the same as mentioned above, shown in EP-A 322,348 in dimethylformamide (DMF) with 4-dimethylaminopyridine (DMAP, 0.2 mmol) as a catalyst for introducing Fmoc-Glu onto said resin via ester bonding.

The side chain-protecting groups in the Fmoc-amino acid derivatives used (Kokusan Kagaku) were as follows: Asp(OBut), Glu(OBut), Thr(But), Ser(But), Tyr(But), Lys(Boc), Arg(Boc) and Cys(Acm). The condensation reaction in each peptide chain extension step was carried out using the corresponding Pfp (pentafluorophenyl)-activated ester (2.5 eq.) in DMF in the presence of 1-hydroxybenzotriazole (HOBT, 0.2 mmol) as a catalyst.

A 20% solution of piperidine in DMF was used for removing each Fmoc group. The coupling reaction in each step was monitored using ninhydrin.

After completion of the final coupling reaction step, the Fmoc group remaining at the N-terminus was eliminated with 20% piperidine in DMF to render the N-terminal amino group free. Then, for removing all other protective groups and for splitting off the peptide from the resin, TFA-thioanisole treatment was performed at room temperature for 3 hours in the presence of m-cresol. The resin was then removed by filtration using a glass filter, the filtrate was concentrated, and the residue was converted into a powder by the addition of ether.

The powder was treated with iodine-methanol at room temperature for 3 hours, which was followed by the addition of 0.5 M aqueous sodium thiosulfate with cooling at 0 ° C. The reaction mixture was concentrated at room temperature.

The residue was collected, dissolved in 0.1 M phosphate buffer (pH 8.6), and applied to a Sephadex G-25 column (product of Pharmacia), elution being carried out with 0.5 N acetic acid for desalting and purification. The peptide after gel filtration was further purified by HPLC.

The purification and recovery by HPLC was performed on a Nucleosil 100-5c18 column (product of M. Nagel; 4.0 x 150 mm) on an acetonitrile concentration gradient of from 10 to 60% in 0.1% TFA at a flow rate of 1 ml/min, each eluate fraction being monitored with an ultraviolet absorption detector at 210 and 260 nm.

EXAMPLE 3

Synthesis of peptide I-b

Fmoc-Gln-OPfp (1 mmol) was reacted with a p-alkoxybenzylalcohol-modified resin (0.2 mmol; product of Kokusan Kagaku Co.; 0.35 meq. OH/g, polystyrene-1% divinylbenzene copolymer) in DMF using DMAP (0.2 mmol) as a catalyst for introducing Fmoc-Glu onto said resin via ester bonding.

The side chain protective groups in the Fmoc-amino acid derivatives used (product of Kokusan Kagaku) were as follows: Asp(OBut), Glu(OBut), Thr(But), Ser(But), Tyr(But), Lys(Boc), Arg(Boc) and Cys(Acm). The condensation reaction in each peptide chain extension step was carried out using the corresponding Pfp-activated ester (2.5 eq.) in DMF in the presence of HOBT (0.2 mmol) as a catalyst. For Fmoc group

elimination, a 20% piperidine in DMF was used. The coupling reaction in each step was monitored using ninhydrin.

After completion of the final coupling reaction step, the Fmoc group remaining at the N terminus was eliminated with a 20% piperidine in DMF to render the N-terminal amino group free. Then, for removing all other protective groups and for splitting off the peptide from the resin, TFA-thioanisole treatment was performed at room temperature for 3 hours in the presence of m-cresol. The resin was then removed by filtration using a glass filter, the filtrate was concentrated, and the residue was converted into a powder by the addition of ether.

The powder was treated with iodine-methanol at room temperature for 3 hours, which was followed by the addition of 0.5 M aqueous sodium thiosulfate solution with cooling at 0°C. The reaction mixture was concentrated at room temperature. The residue was collected, dissolved in 0.1 M phosphate buffer (pH 8.6), and applied to a Sephadex G-25 column (product of Pharmacia), elution being carried out with 0.5 N acetic acid for desalting and purification. The peptide after gel filtration was further purified by HPLC.

The purification and recovery by HPLC was performed on a Nucleosil 100-5c18 column (M. Nagel; 4.0 x 150 mm) on an acetonitrile concentration gradient of from 10 to 60% in 0.1% TFA at a flow rate of 1 ml/min, each eluate fraction being monitored by ultraviolet absorption detector at 210 and 260 nm. The retention time of the desired peptide (peptide I-b) was 12.9 minutes.

The results of amino acid analysis of the peptide obtained were as shown below and in agreement with the calculated values.

Asp (2) 2.01, Thr (2) 1.94, Ser (1) 0.94, Glu (2) 1.99, Pro (2) 2.01, Gly (4) 4.01, Ala (1) 1.01, 1/2 Cystine (2) 1.81, Val (1) 0.95, Met (1) 0.98, Tyr (2) 1.95, Phe (2) 2.01, Lys (1) 1.00, Arg (2) 2.02, $NH_3$ (3) 3.23. (Values in the parentheses are calculated values.)

Peptide I-a of the present invention shows a JH esterase activity repressing activity against last instar larvae of Pseudaletia separata and has a pupation retarding effect, namely a development controlling effect, on said larvae.

The last instar larvae treated with peptide I-a show a decreased food intake and can no longer grow. Peptide I-a thus shows its effect in a delayed manner and yet can be expected to be effective also as an insecticide.

Peptide I-b of the present invention has a very strong lethal effect on last instar larvae of Pseudaletia separata and Spodoptera litura. Thus, peptide I-b is expected as an insecticide effective at low doses.

The peptide [I] of the invention displays a good effect on Lepidopteran insects, for example, Pyralid moths (Pyralidae) such as rice stem borer (Chilo suppressalis), rice leafroller (Cnaphalocrocis medinalis) and Indian meal moth (Plodia interpunctella); Noctuidae such as tobacco curworm (Spodoptera litura), rice armyworm (Pseudaletia separata, cabbage armyworm (Mamestra brassicae), beet semi-looper (Autographa nigrisigna), turnip cutworm (Agrotis segetum) , black cutworm (Agrotis ipsilon) and Heliothis spp.; Pieridae such as common cabbageworm (Pieris rapae crucivora); tortricid moths (Tortricidae) such as Adoxophyes spp. and Grapholita spp.; Carposinidae ; lyonetiid moths (Lyonetiidae); leafblotch miners (Gracillariidae); gelechiid moths (Gelechiidae), tussock moths (Lymantriidae); diamondback moth (Plutella xylostella); clothes moths (Tineidae) such as casemaking clothes moth (Tinea translucens) and webbing clothes moth (Tineola bisselliella), etc. Peptide [I], in particular, displays an excellent effect on larvae.

When the peptide [I] of the invention was used as an active ingredient of an insect pest controlling agent, the peptide was generally used in the form of formulations such as emulsion, water dispersible powder, flowable agent containing oil-in-water suspension and oil-in-water emulsion, granule, dust, ULV agent, poison bait with mixing a solid carrier, a liquid carrier or a bait or adding a surface active agent or other additives for formulations.

Further, a transformant prepared by integrating genes which code the peptides into suitable vectors (e.g. a nuclear polyhedrosis virus) or microorganisms (e.g. Psedomonas spp.) can be used in suitable formulations as mentioned above.

As a solid carrier utilized in preparing formulations, exemplified are fine powders or granular materials, such as crays (kaolin cray, diatomaceous earth, synthetic water-containing silicone oxide, bentonite, fubasami cray, acidic terra alba, etc.), talcs, ceramic and other inorganic minerals (sericite, quartz, sulfur, active carbon, calcium carbonate, silica hydrate, etc.), chemical fertilizers (ammonium sulfate, ammonium phosphate, ammonium nitrate, urea, ammonium chloride etc.). As a liquid carrier, exemplified are water, alcohols (methanol, ethanol, etc.), ketones (acetone, methylethylketone etc.), aromatic hydrocarbones (benzene, toluene, xylene, ethylbenzene, methylnaphthalene, etc.), aliphatic hydrocarbons (hexane, cyclohexane, kerosene, gas oil, etc.), esters (ethyl acetate, butyl acetate, etc.), nitriles (acetonitrile, isobutylnitrile, etc.), ethers (diisopropylether, dioxane, etc.), acid amides (N,N-dimethylformamide, N,N-dimethylacetoamide, etc.), hydrocarbon halides (dichloromethane, trichloroethane, etc.), dimethylsulfoxide,

plant oils such as soy bean oil and cotton seed oil.

As a surface active agent, exemplified are alkylsulfuric acid esters, alkylsulfonate, alkylarylsulfonate, alkylarylethers and polyoxyethylene-modified alkylarylether, polyethyleneglycolethers, polyhydric alcohol esters, sugar alcohol derivatives.

As ajuvants for formulations such as adhesive agent and dispersing agent, exemplified are casein, gelatin, polysaccharides (starch powder, gum arabic, cellulose derivatives, alginic acid, etc.), lignin derivatives, bentonite, saccharides, synthetic watersoluble polymers (polyvinylalcohol, polyvinyl pyrrolidone, polyacrylic acids, etc.).

As stabilizers, exemplified are acid isopropyl phosphate (PAP), 2,6-di-tert-butyl-4-methylphenol (BHT), a mixture of 2-tert-butyl-4-methoxyphenol and 3-tert-bytyl-4-methoxyphenol (BHA), plant oil, mineral oil, surface active agent, fatty acid, and fatty acid ester.

As bases of a poison bait, exemplified are bait constituents such as grain powder, plant essential oil, sugar, crystal cellulose, antioxidants such as dibutylhydroxytoluene, nordihydroguaiaretic acid, preservatives such as dehydroacetic acid, such as powdered capsicum, attractive spices such as cheese spice, and onion spice.

Formulations of a flowable agent (oil-in-water suspension or oil-in-water emulsion) can be generally obtained by microscopically dispersing the peptide in the range of 1 to 75 % in water containing a dispersing agent in the range of 0.5 to 15 %, a suspension adjuvant such as a compound giving characteristics of protective colloid or thixotropy in the range of 0.1 to 10 % and a suitable adjuvant (ex., antifoamer, anticorrosive agent, stabilizer, sticker, infiltration adjuvant, cryoprotective compound, antimicrobial agent, antimycotic agent etc.) in the range of 0 to 10 %. A water-in-oil emulsion can be prepared by using a oil in which the peptide is substantially insoluble in place of water. Geranine, casein, gums, cellulose esters, polyvinylalcohol and the like are exemplified as a protective colloid. Bentonite, magnesium aluminum silicate, xanthane gum, polyacrylic acids and the like are exemplified as a compound giving a characteristics of thixotropy to the peptide.

The formulations thus obtained are used as it is or diluting with water and the like. The formulations can be used mixing with other additives such as insecticide, miticide, nematicide, soil insect pest controlling agent, bacteriocide, herbicide, plant growth regulator, synergist, fertilizer or soil conditioner, or simultaneously without mixing them with the additives.

When the peptide [I] of the invention is used as an active ingredient of an insect pest controlling agent for agricultural use, the amount of application is generally in the range of 0.01 to 100 g per ten ares. The application concentration of the peptide is in the range of 0.5 to 500 ppm when a flowable agent and the like diluted with water is applied. A granule, dust and the like are used as they are without mixing. When used as an insect pest controlling agent for sanitary use, water dispersible powder, flowable agent, water dispersible agent and the like is applied after the formulations are diluted to the concentration of the peptide in the range of 0.5 to 500 ppm, ULV agent, poison bait and the like being applied as they are.

Those application amount and application concentration are varied with conditions such as a kind of formulation, application stage, application place, application method, a species of insect pest, extent of damage and can be increased or decreased in spite of the above range.

Formulation Examples are shown below. The parts and percentages are all by weight in the Examples.

Formulation Example 1

Water dispersible powder

20 parts of peptide I-a or I-b were added to the mixture consisting of 4 parts of sodium lauryl sulfate, 2 parts of calcium lignin sulfonate, 20 parts of synthetic water-containing silicone oxide fine powder and 54 parts of diatomaceous earth. The mixture was stirred and mixed with a juice mixer. 20 % of water dispersible powders of peptide I-a and I-b were obtained respectively.

Formulation Example 2

Granule

To 5 parts of peptide I-a or I-b were added 5 parts of synthetic water containing silicone oxide fine powder, 5 parts of sodium dodecylbenzenesulfonate, 30 parts of bentonite and 55 parts of cray, the mixture being sufficiently stirred and mixed. Adequate amount of water was then added to the mixture and the mixture was stirred, granulated with a granulator and dried with aeration. 5 % of granules of peptide I-a and

I-b were obtained respectively.

Formulation Example 3

Dust

Peptide I-a or I-b was dissolved in an adequate amount of acetone. To the solution were added 5 parts of synthetic water-containing silicon oxide, 0.3 parts of PAP and 93.7 parts of clay, and the mixture being stirred and mixed with a juice mixer. After evaporation of acetone, dusts of peptide I-a and I-b were obtained.

Formulation Example 4

Flowable agent

20 parts of peptide I-a or I-b and 1.5 parts of sorbitan trioleate were mixed with 28.5 parts of water solution containing 2 parts of polyvinylalcohol. The mixture was finely pulverized (particle size not more than 3 μm) with a sand grinder. To the fine powder was added 40 parts of water solution containing 0.05 parts of xanthane gum and 0.1 part of magnesium aluminum silicate, and then added 10 parts of propyleneglycol. After stirring and mixing the mixture, water suspensable powders of 20 % of peptide I-a or I-b were obtained respectively.

The present invention is described in more detail by the following examples for biological activity.

EXAMPLE 4

Physiological activity of peptide I-a

To examine the effect of peptide I-a on the growth of last instar larvae of Pseudaletia separata, last instar larvae of P. separata were injected with 7 picomoles of peptide I-a for consecutive three days (day 0, day 1 and day 2). They were reared on an artificial diet and the weight of each larva was checked until day 3. The result is shown in Fig. 4.

Fig. 4 graphically shows the body weight change in last instar larvae of Pseudaletia separata injected with peptide I-a. The open circles (○) indicate the body weights of the last instar larvae injected with 7 picomoles of peptide I-a on day 0, day 1 and day 2 while the closed circles (●) indicate the body weights of last instar larvae injected with Ringer's solution on day 0, day 1 and day 2.

As shown in Fig. 4, the larvae injected with peptide I-a showed only slight increases in body weight while, in a control group, the body weight increased almost linearly from day 0 until day 3, the body weight on day 3 being about 2.5 times as much weight as that on day 0.

EXAMPLE 5

JH esterase repressing activity of peptide I-a

The purified peptide I-a was diluted with Ringer's solution and injected, at a predetermined dose, into unparasitized last instar larvae of Pseudaletia separata on day 1 and day 2. The larvae were further reared on an artificial diet. On day 3, hemolymph was prepared from the larvae and centrifuged (500 x g, 4°C, 5 minutes). The supernatant thus collected was subjected to JH esterase activity assay.

(10-$^3$H)JH I was used as the substrate for activity assay and the JH-metabolizing activity was estimated based on the proportion of JH acid to the total product formed under the action of JH esterase. The result is shown in Fig. 5.

Fig. 5 graphically shows the results of JH esterase activity assay of plasma samples collected, on day 3, from last instar larvae of Pseudaletia separata injected with different doses of peptide I-a on day 1 and day 2 of the last instar.

As shown in Fig. 5, peptide I-a repressed the JH esterase activity in a dose dependent manner.

EXAMPLE 6

Insect development controlling activity

The purified peptide I-a was dissolved with Ringer's solution and injected, at a dose of 7 picomoles, into unparasitized last instar larvae of Pseudaletia separata on day 1 and day 2. Then the growth stage (larva, prepupa, pupa, or dead) of each larva was checked at 24-hour intervals and the number of individuals in each stage was recorded. Control larvae were injected with Ringer's solution alone.

The result is shown in Table 2.

In larvae treated with peptide I-a, the prepupal stage came later by 2 days and pupation was later by more than 2 days than in control larvae, as shown below in Table 2.

Table 2 Influence of peptide I-a on pupation of unparasitized last instar larvae of <u>Pseudaletia separata</u>

| | No. of hosts examined | Stage* | Day of last instar larvae 1 | 2 | 3 | 4 | 5 | 6 | 7 | 8 | 9 | 10 | 11 | 12 |
|---|---|---|---|---|---|---|---|---|---|---|---|---|---|---|
| Treated | 17 | L | 17 | 17 | 17 | 17 | 17 | 17 | 14 | 5 | 1 | | | |
| | | PP | | | | | | | 3 | 11 | 11 | 4 | 3 | |
| | | P | | | | | | | | | 3 | 11 | 12 | 15 |
| | | D | | | | | | | | 1 | 2 | 2 | 2 | 2 |
| Control | 16 | L | 16 | 16 | 16 | 16 | 16 | | | | | | | |
| | | PP | | | | | | 16 | 16 | | | | | |
| | | P | | | | | | | | 15 | | | | |
| | | D | | | | | | | | 1 | | | | |

*L, larva; PP, prepupa; P, pupa; D, dead.

EXAMPLE 7

Physiological activity of peptide I-b

To examine the insecticidal activity of peptide I-b against last instar larvae of Pseudaletia separata, a solution of peptide I-b in Ringer's solution was injected, at a dose of 4 picomoles, into day 0 last instar larvae of P. separata on day 0. The result is shown in Table 3.

Table 3

| Treatment | Mortality (%) after 48 hours |
|---|---|
| Peptide I-b (7 pmol) | 100 |
| Control | 0 |

EXAMPLE 8

Physiological activity of peptide I-b

To examine the paralyzing activity of peptide I-b against last instar larvae of Spodoptera litura, a solution of peptide I-b in Ringer's solution was injected, at a dose of 36 picomoles, into last instar larvae of S. litura on day 0. The result is shown in Table 4.

Table 4

| Treatment | paralysis (%) after 10 min | |
|---|---|---|
| Peptide I-b (36 pmol) | | 75.0 |
| Control | | 0 |

Sequence Listing

SEQ ID NO:1

SEQUENCE TYPE: amino acids

SEQUENCE LENGTH: 25 amino acids

TOPOLOGY: topology unknown to applicant

MOLECULE TYPE: peptide

ORIGINAL SOURCE: Pseudaletia Separata

FEATURES

disulfide bridge between $Cys^7$ and $Cys^{19}$

Glu Asn Phe Ser Gly Gly Cys Val Ala Gly Tyr Met Arg Thr Pro Asp
1 5 10 15

Gly Arg Cys Lys Pro Thr Phe Tyr Gln
20 25

SEQ ID NO:2

SEQUENCE TYPE: amino acids

SEQUENCE LENGTH: 25 amino acids

TOPOLOGY: topology unknown to applicant

MOLECULE TYPE: peptide

ORIGINAL SOURCE: Pseudaletia Separata

FEATURES

C-terminus converted to $CONH_2$

disulfide bridge between $Cys^7$ and $Cys^{19}$

Glu Asn Phe Ser Gly Gly Cys Val Ala Gly Tyr Met Arg Thr Pro Asp
1 5 10 15

Gly Arg Cys Lys Pro Thr Phe Tyr Gln
20 25

## Claims

**1.** A compound of the formula [I]:

Glu-Asn-Phe-Ser-Gly-Gly-Cys-Val-Ala-Gly-

Tyr-Met-Arg-Thr-Pro-Asp-Gly-Arg-Cys-Lys- [I]

Pro-Thr-Phe-Tyr-Gln-R

wherein R is an amino group or a hydroxy group.

2. A composition for controlling insect development and/or insecticide which comprises, as an active ingredient, the compound according to claim 1 in an amount effective for controlling insect development and/or insecticide, and an inert carrier or diluent.

3. A method for controlling insect development and/or insecticide which comprises applying the compound according to claim 1 in an amount effective for controlling insect development and/or insecticide to the insects.

4. The method according to claim 3, wherein the insects are Lepidoptera.

5. Use of the compound according to claim 1 as controlling agent for insect development and/or insecticide.

FIG. 1

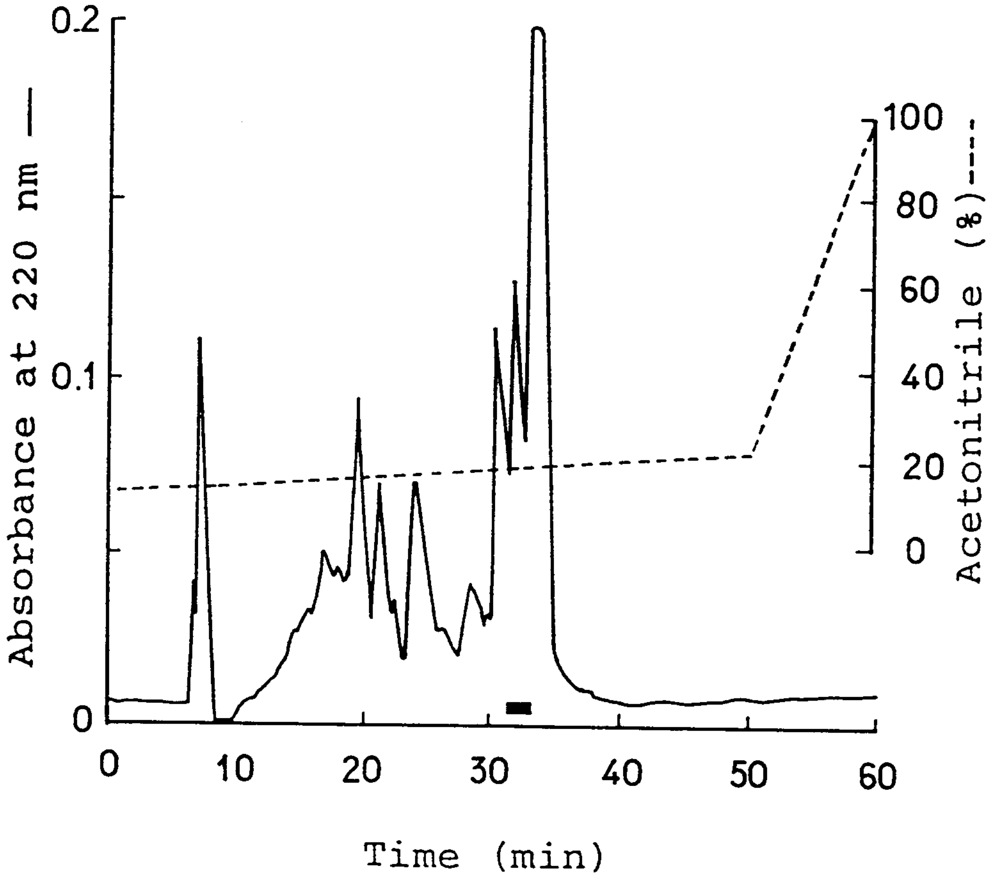
0.2
0.1
0
Absorbance at 220 nm —
100
80
60
40
20
0
Acetonitrile (%)-----
0
10
20
30
40
50
60
Time (min)

FIG. 2

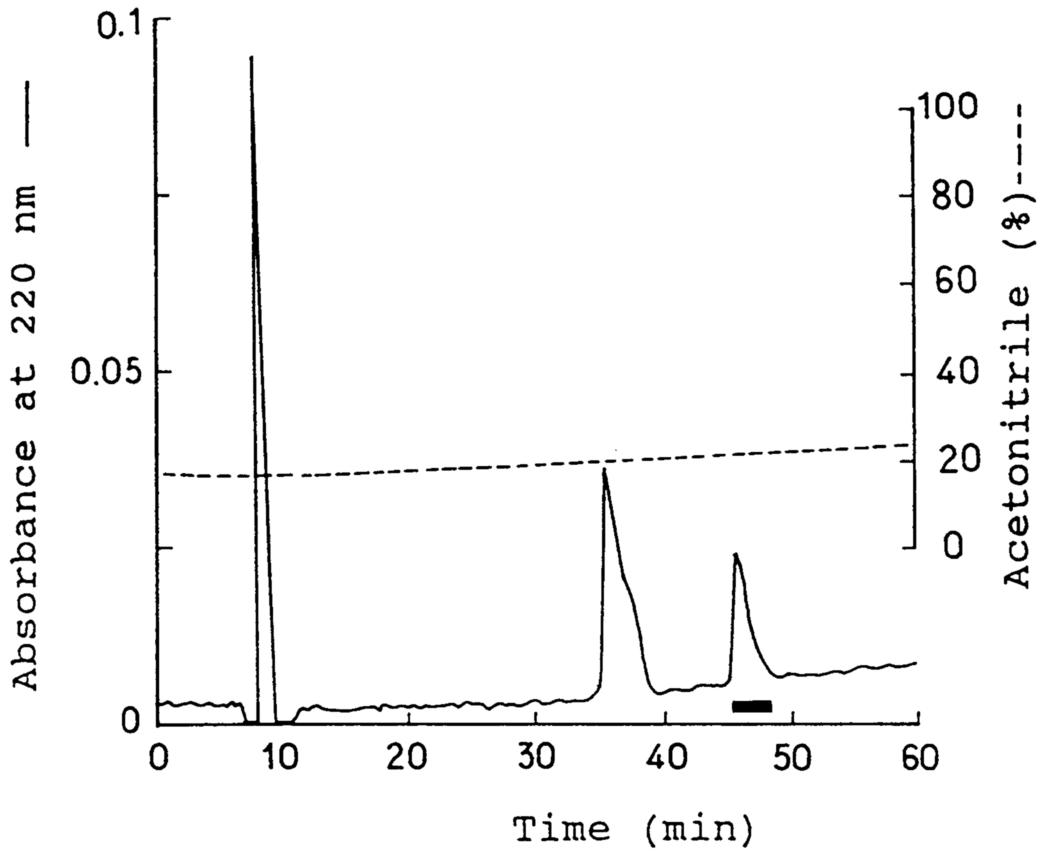
0.1
0.05
0
Absorbance at 220 nm —
100
80
60
40
20
0
Acetonitrile (%)----
0
10
20
30
40
50
60
Time (min)

FIG. 3

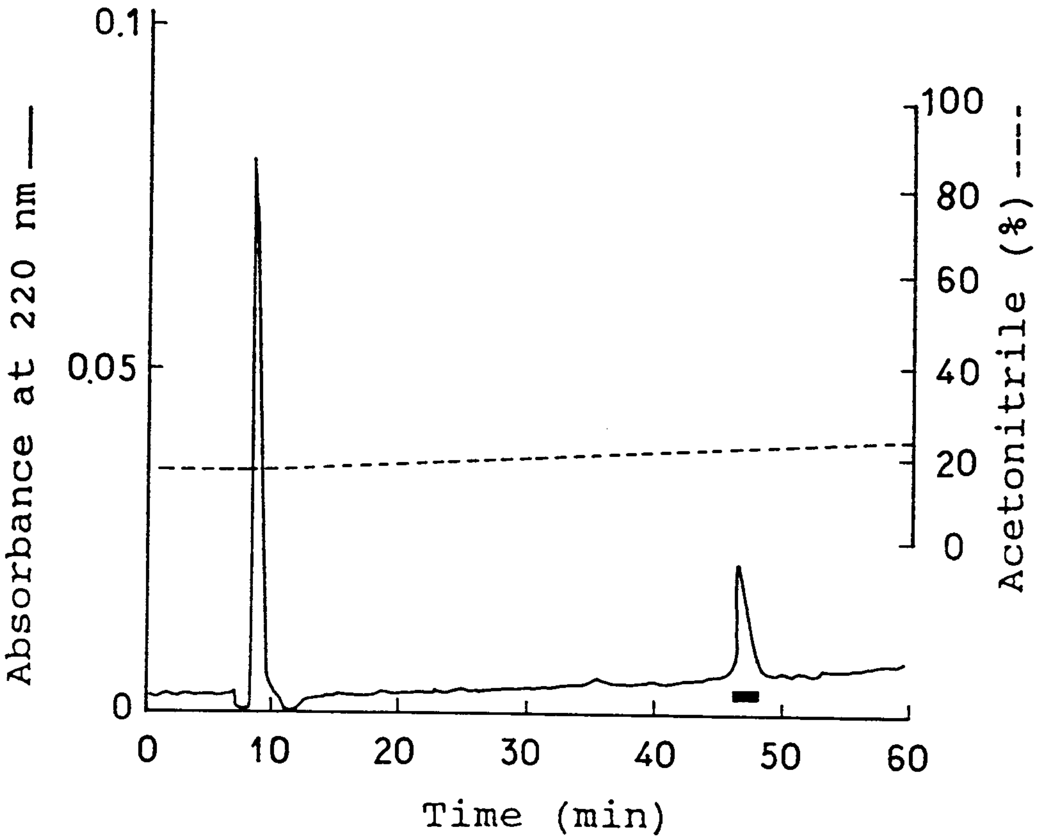
0.1
0.05
0
Absorbance at 220 nm
100
80
60
40
20
0
Acetonitrile (%)
0
10
20
30
40
50
60
Time (min)

FIG. 4

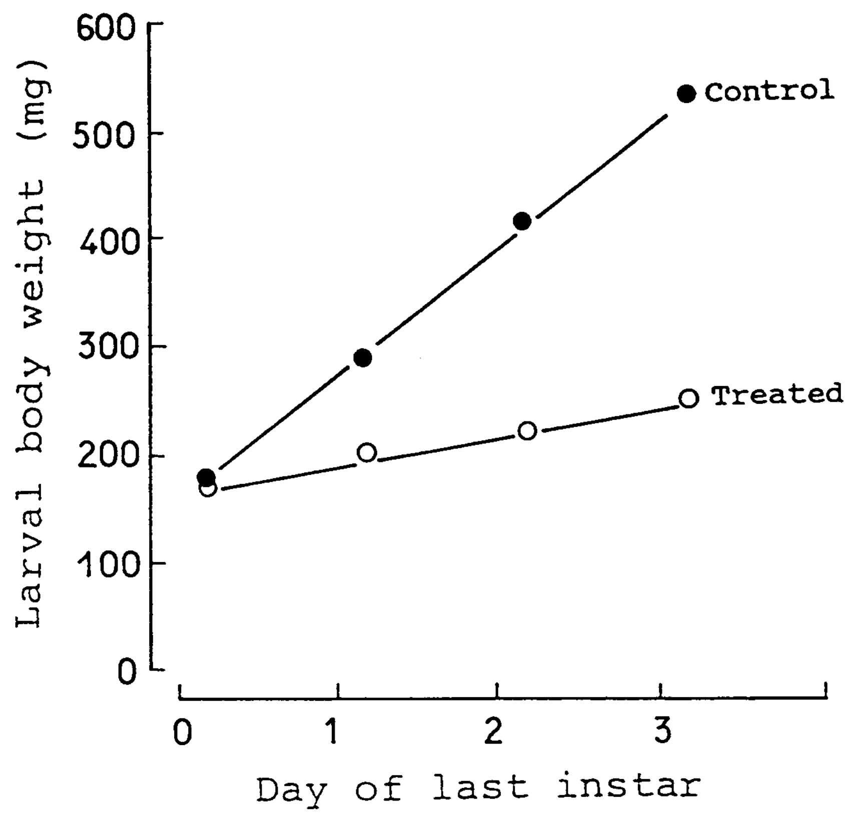
Larval body weight (mg)
600
500
400
300
200
100
0
Control
Treated
0
1
2
3
Day of last instar

FIG. 5

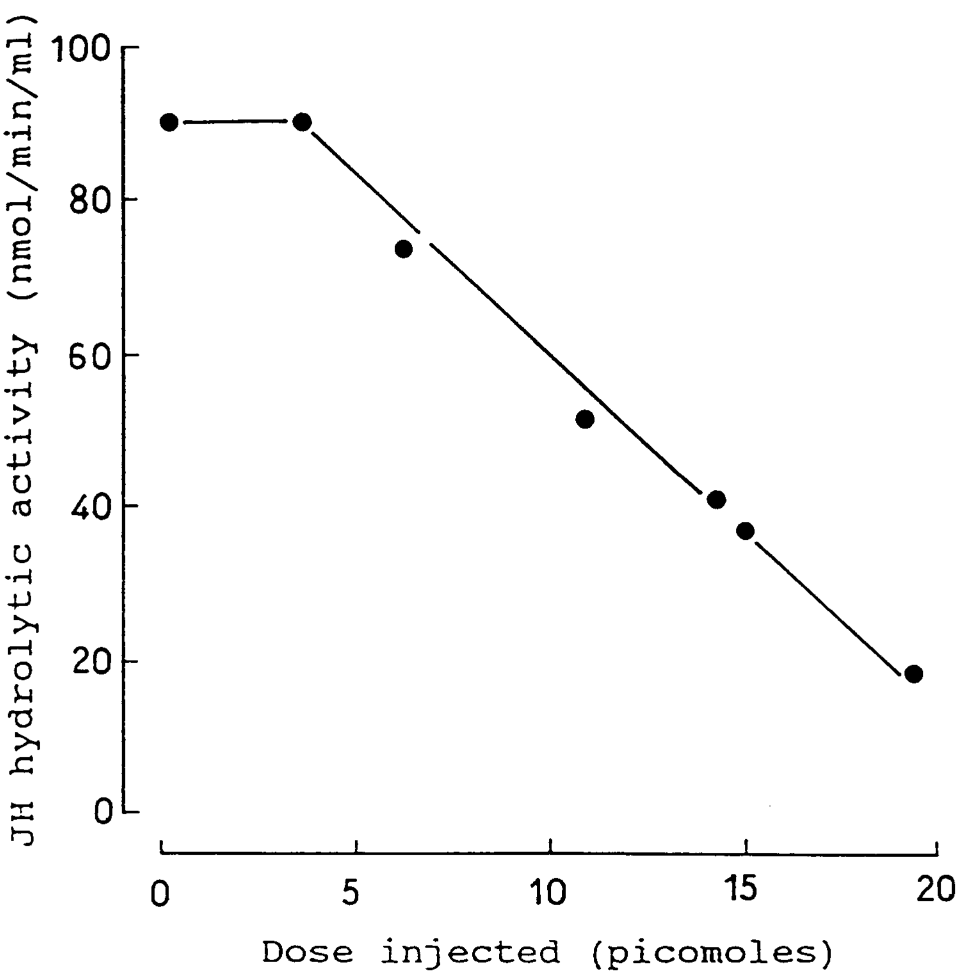
JH hydrolytic activity (nmol/min/ml)
100
80
60
40
20
0
0
5
10
15
20
Dose injected (picomoles)

European Patent Office

# EUROPEAN SEARCH REPORT

Application Number

EP 92101084.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 113, no. 7, August 13, 1990, Columbus, Ohio, USA HAYAKAMA, YOICHI "Juvenile hormone esterase activity repressive factor in the plasma of parasitized insect larvae.", page 413, column 2, abstract-no. 56 114j & J. Biol. Chem. 1990, 265(19), 10813-16 -- | 1-5 | C 07 K 7/64 |
| X | CHEMICAL ABSTRACTS, vol. 113, no. 9, August 27, 1990, Columbus, Ohio, USA TANAKA, TOSHIHARU et al. "Ultrastructural and functional maturation of teratocytes of Apanteles kariyai.", page 486, column 1, abstract-no. 75 251t & Arch. Insect Biochem. Physiol. 1990, 13(3-4), 187-97 -- | 1-5 | TECHNICAL FIELDS SEARCHED (Int. Cl.5)<br>C 07 K 7/00<br>C 12 P 21/00 |
| A | CHEMICAL ABSTRACTS, vol. 113, no. 9, August 27, 1990, Columbus, Ohio, USA RAINA, ASHOK K. et al. "Peptides stimulating sex pheromone production and melanization in moths.", page 273, column 1, abstract-no. 73 040z & US-A-354 326 (US Dept. of Agriculture), September 15, 1989 -- | 1-5 | |

The present search report has been drawn up for all claims

| Place of search | Date of completion of the search | Examiner |
|---|---|---|
| VIENNA | 29-04-1992 | SCHARF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

-2-

EP 92101084.9

| DOCUMENTS CONSIDERED TO BE RELEVANT | | | |
| --- | --- | --- | --- |
| Category | Citation of document with indication, where appropriate, of relevant passages | Relevant to claim | CLASSIFICATION OF THE APPLICATION (Int. Cl.5) |
| D,A | PROCEEDINGS OF THE NATIONAL ACADEMY OF SCIENCES OF THE UNITED STATES OF AMERICA, vol. 83, no. 16, August 1986, HIROMICHI, NAGASAWA et al. "Amino acid sequence of a prothoracicotropic hormone of the silkworm Bombyx mori", pages 5840-5843 * Abstract * ---- | 1-5 | |
| | | | TECHNICAL FIELDS SEARCHED (Int. Cl.5) |

The present search report has been drawn up for all claims

| Place of search | Date of completion of the search | Examiner |
| --- | --- | --- |
| VIENNA | 29-04-1992 | SCHARF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)